# EUROPEAN PATENT SPECIFICATION

(11) **EP 0 801 542 B1**
(45) Date of publication and mention of the grant of the patent: **05.06.2002**
(21) Application number: 96943023.0
(22) Date of filing: 24.10.1996
(51) Int. Cl.: A61B 17/64

(54) **DEVICE FOR OSTEOSYNTHESIS**
OSTEOSYNTHESEHILFSMITTEL
APPAREIL POUR L'OSTEOSYNTHESE

(30) Priority: 26.10.1995 DE 29516910 U
(43) Date of publication of application: 22.10.1997
(73) Proprietor: Pennig, Dietmar, Dr., 50935 Köln (DE)
(72) Inventor: Pennig, Dietmar, Dr., 50935 Köln (DE)
(74) Representative: Botti, Mario
(86) International application number: EP9604630
(87) International publication number: WO9717031

(56) References cited:
- EP-A- 0 490 812
- WO-A-96/12443
- DE-C- 4 231 443
- DE-U- 9 108 566
- GB-A- 2 229 096

## Description

### Technical Field

The invention relates to a device for osteosynthesis. According to one preferred aspect of the invention, there is disclosed a device for osteosynthesis which is particularly useful for fixing fractured ends of curved relatively small bones, for promoting the healing of such bones.

### Background Art

Known osteosynthesis devices, which in particular are intended to fix curved and relatively small bones, include support plates which carry fixation means and which are interconnected by a ball joint. In such osteosynthesis devices, the greatest possible degree of angular positioning of the two retainer or support plates relative to one another is appropriately provided according to the circumstances. On the other hand, however, once these osteosynthesis devices are in their set position, it must be ensured that any involuntary shifting is absolutely excluded. In the previously-known osteosynthesis devices, this is achieved by double-ball joint connection which is securable by fixing means. Examples of these conventional osteosynthesis devices are described in US Patent No. 4.988,349, the disclosure of which is incorporated herein by reference.

While conventional osteosynthesis devices work admirably, there exists the need to provide improved osteosynthesis devices which achieve improved functionality and results.

An improvement in this field has been represented by the clamping coupling for fixing bone screws and the like disclosed in DE-U-91 08 566, which incorporates a rotary joint between the bone-screw fixing region of the coupling and a ball-joint connection region of the coupling.

### Disclosure of the Invention

In accordance with one preferred aspect of invention, there is provided an osteosynthesis device of the type in question, which involves the use of only one ball head of a ball joint, while the other support plate is connected via a joint mobile in one direction, and which has the features disclosed in appended claim 1.

In the device according to the invention, a ball joint with a ball head is connected with one support plate of an osteosynthesis device, while a hinge-like joint which is securable in only one plane is connected with the other support plate of the osteosynthesis device. By virtue of the fact, however, that this joint is mounted on a rotatable pin, and which is thus rotatable about an axis extending transversely to the joint axis, a relatively large degree of mobility and of facility of alignment of the joint is provided, and greater strength is still achieved in one plane, as the actual hinge-like joint is movable in only one plane, so that a great degree of strength is achieved in the other plane.

The technical characteristics of the invention may be understood from the following detailed description of some possible examples of preferred embodiments thereof with reference to the drawings.

### Brief Description of Drawings

Fig. 1 is a detail perspective view of an osteosynthesis device for promoting the healing of fractured bones which includes an interconnection in accordance with one preferred embodiment of the invention of two support plates of the device; and
Fig. 2 is a partial cross-section view of the device of Fig. 1 taken along plane II-II of Fig. 1.

### Best Mode for Carrying Out the Invention

The numerals 1 and 2 refer in the drawings to respective support plates of the osteosynthesis device, serving to secure suitable means for fixation of the device to a fractured bone. Supports plates 1 and 2 may support fixation means of the type described in US Patent No. 4,988,349, including clamping members adjustably screwed to sliding blocks slidingly connected in slide guides 1a and 2a respectively of support plates 1 and 2, and including bone screws clamped by the clamping members. The precise type of fixation means carried by support plates 1 and 2 may be any one of many, as understood by a skilled person in the art.

A ball joint 3 is connected in a known manner to support plate 2. The ball joint 3 includes a ball head 4, and connected to and extending from the ball head 4 there is a rigid distance piece 5. In particular, the ball joint 3 includes a cylindrical element 3a which is connected to support plate 2 and which includes a threaded portion at its outer surface arranged distally from support plate 2. The ball joint 3 further includes a locking nut 3b having an internal seat in which ball 4 is lodged, and having an internally threaded portion such that locking nut 3b is screwed onto cylindrical element 3a. In a non-locked position of locking nut 3b, ball 4 is free to rotate in the internal seat of locking nut 3b, and when the locking nub 3b is tightened ball 4 is pushed to engage against a corresponding seat portion of cylindrical element 3a so as to block the movement of the ball 4, in a known manner.

In conventional devices, distance piece 5 would be connected at its end opposite ball 4 to another ball of a double-ball joint, which other ball would be connected to support plate 1 in a manner completely similar to the connection of ball 4 to support plate 2 by means of joint 3.

In accordance with the invention however, distance piece 5 opens into a joint 6, which is pivotable about the pivot axis x-x. A securing screw 8 serves to block this pivotal movement.

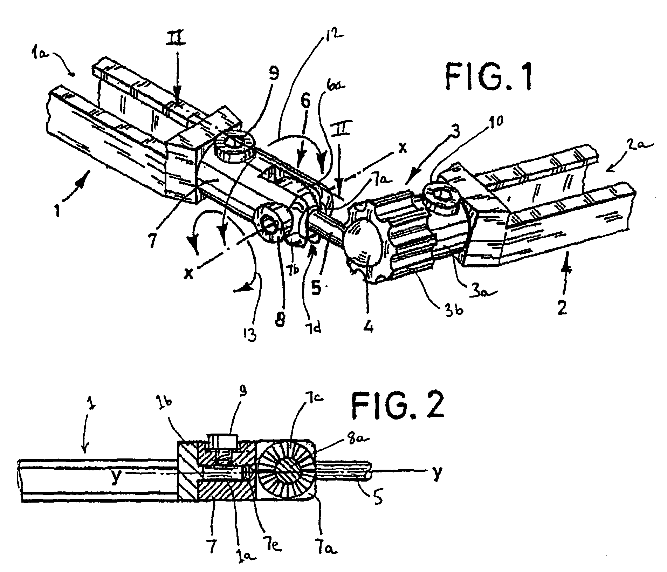

In particular, the joint 6 includes a locking portion 6a rigidly connected to distance piece 5 and supported by a carrier 7 in a manner such that locking portion 6a may rotate into different locked positions only about axis x-x. In particular, carrier 7 includes a pair of lateral fork portions 7a and 7b each having concave inner surfaces 7c which together form a seat for locking portion 6a. Radial teeth are provided on inner surfaces 7c of lateral fork portions 7a and 7b for improving the locking grip on locking portion 6a, as seen in Fig. 2 in which locking portion 6a has been omitted. A stem 8a of securing screw 8 extends through fork portion 7b and through a through channel in locking portion 6a, and is screwed into the opposite fork portion 7a, so that securing screw 8 may be adjusted to mutually space or bring closer together the fork portions 7a and 7b for allowing rotation of locking portion 6a about axis x-x or alternatively for blocking the movement of locking portion 6a. A channel 7d is provided between fork portions 7a and 7b which extends in plane II-II and outside of which the distance piece 5 extends, for substantially permitting rotation of locking portion 6a only about axis x-x.

Carrier 7 is rotatable about the longitudinal axis y - y of support plate 1 (Fig. 2) with respect to support plate 1, and can be secured and locked in place with respect to support plate 1 by means of a fixing screw 9. In particular, a pin 1a extends from an end plate 1b of support plate 1 and is lodged in a axial cavity 7e of carrier 7 such that carrier 7 is rotatable about axis y-y with respect to support plate 1. Fixing screw 9 is screwed into the lateral surface of carrier 7 and may be rotated so as to engage pin 1a sufficiently to block the rotation of carrier 7. Cylindrical element 3a is rotatably connected to support plate 2 in a similar manner about the longitudinal axis of support plate 2, and may be selectively locked in place by means of fixing screw 10.

By means of the arrangement of parts herein described, rotary movements about the curved arrows 12 and 13 (Fig. 1) are possible, and yet good securement and blocking of the overall arrangement can be achieved.

## Claims

1. An osteosynthesis device comprising:
a pair of support plates (1, 2) for carrying fixation elements for fixing the device to a bone;
a ball joint (3) connected to a first one (2) of said support plates and including a rotatable ball element (4) releasably lockably supported in the ball joint;
a hinge joint (6) connected to a second one (1) of said support plates; and
a rigid distance piece (5) which is rigidly connected to said ball element and which is connected to said hinge joint such that said distance piece (5) is rotatably positionable about only a single axis (x-x) of the hinge joint into selected locked positions, wherein said hinge joint (6) is rotatably connected to said second support plate about a rotation axis (y-y) and wherein said rotation axis (y-y) extends essentially perpendicularly to said single axis (x-x).

2. An osteosynthesis device according to claim 1, wherein said ball joint (3) is connected to said first support plate about a rotation axis.

3. An osteosynthesis device according to claim 1, wherein said hinge joint (6) comprises:
a locking portion (6a) rigidly connected to said distance piece;
a carrier (7) connected to said second support plate at a first end thereof and comprising a pair of lateral fork portions (7a, 7b)arranged at a second end thereof, said fork portions defining an expanding and contracting seat in which said locking portion is accomodated; and
means (8) for releasably contracting said seat of said fork portions for releasably blocking said locking portion in selected releasably blocked positions;

4. An osteosynthesis device according to claim 3, wherein said fork portions define a channel (7d) extending in a plane which is perpendicular to said single axis (x-x), said distance piece extending outside said channel.

## Patentansprüche

1. Osteosynthese-Vorrichtung, aufweisend:
ein Paar Halterungsplatten (1, 2) zum Tragen von Fixierelementen zum Fixieren der Vorrichtung an einem Knochen;
ein Kugelgelenk (3), das mit einer ersten (2) der Halterungsplatten verbunden ist und ein drehbares Kugelelement (4) aufweist, das in dem Kugelgelenk lösbar verriegelbar gehaltert ist;
ein Klapp-Gelenk (6), das mit einer zweiten (1) der Halterungsplatten verbunden ist; und
ein starres Distanzstück (5), das mit dem Kugelelement starr verbunden ist und das mit dem Klapp-Gelenk derart verbunden ist, dass das Distanzstück (5) nur um eine einzige Achse (x-x) des Klapp-Gelenks schwenkbar in ausgewählten verriegelten Positionen positionierbar ist,
wobei das Klapp-Gelenk (6) mit der zweiten Halterungsplatte um eine Drehachse (y-y) drehbar verbunden ist und wobei die Drehachse (y-y) im Wesentlichen rechtwinklig zu der genannten einzigen Achse (x-x) verläuft.

2. Osteosynthese-Vorrichtung nach Anspruch 1,
wobei das Kugelgelenk (3) mit der ersten Halterungsplatte über eine Drehachse verbunden ist.

3. Osteosynthese-Vorrichtung nach Anspruch 1,
wobei das Klapp-Gelenk (6) Folgendes aufweist:
einen Verriegelungsbereich (6a), der mit dem Distanzstück starr verbunden ist;
einen Träger (7), der an einem ersten Ende mit der zweiten Halterungsplatte verbunden ist und an dessen zweitem Ende ein Paar seitlicher Gabelbereiche (7a, 7b) angeordnet ist, wobei die Gabelbereiche einen erweiterbaren und verengbaren Sitz bilden, in dem der Verriegelungsbereich aufgenommen ist; und
eine Einrichtung (8) zum lösbaren Verengen des Sitzes der Gabelbereiche zum lösbaren Blockieren des Verriegelungsbereichs in ausgewählten, lösbar blockierten Positionen.

4. Osteosynthese-Vorrichtung nach Anspruch 3,
wobei die Gabelbereiche einen Kanal (7d) bilden, der in einer Ebene verläuft, die rechtwinklig zu der genannten einzigen Achse (x-x) ist,
wobei sich das Distanzstück außerhalb des Kanals erstreckt.

## Revendications

1. Dispositif d'ostéosynthèse, comprenant :
- une paire de plaques de support (1, 2) pour porter des éléments de fixation afin de fixer le dispositif sur un os ;
- une rotule (3) connectée à une première (2) desdites plaques de support et incluant un élément sphérique (4), susceptible de tourner, supporté de façon verrouillable et déverrouillable dans la rotule ;
- une articulation (6) connectée à une deuxième (1) desdites plaques de support ; et
- une pièce rigide de distanciation (5) qui est connectée rigidement audit élément sphérique et qui est connectée à ladite articulation, de manière à ce que ladite pièce de distanciation (5) puisse être positionnée, de manière à pouvoir tourner, autour d'un axe unique (x-x) de l'articulation, en des positions verrouillées sélectionnées, ladite articulation (6) étant connectée, de façon à pouvoir tourner, à ladite deuxième plaque de support autour d'un axe de rotation (y-y) et ledit axe de rotation (y-y) s'étendant sensiblement perpendiculairement audit axe unique (x-x).

2. Dispositif d'ostéosynthèse selon la revendication 1, dans lequel ladite rotule (3) est connectée à ladite première plaque de support, autour d'un axe de rotation.

3. Dispositif d'ostéosynthèse selon la revendication 1, dans lequel ladite articulation (6) comprend :
- une partie de verrouillage (6a) connectée rigidement à ladite pièce de distanciation ;
- un support (7) connecté à ladite deuxième plaque de support par une première extrémité de celui-ci et comprenant une paire de parties latérales en fourche (7a, 7b) disposées à une deuxième extrémité de celui-ci, lesdites parties en fourche définissant un siège s'expansant et se contractant, dans lequel est accommodée ladite partie de verrouillage ; et
- des moyens (8) pour contracter, de façon libérable, ledit siège desdites parties en fourche, afin de bloquer, de façon libérable, ladite partie de verrouillage en des positions sélectionnées de blocage libérable.

4. Dispositif d'ostéosynthèse selon la revendication 3, dans lequel lesdites parties en fourche définissent un canal (7d) s'étendant dans un plan perpendiculaire audit axe unique (x-x), ladite pièce de distanciation s'étendant à l'extérieur dudit canal.
